# EUROPEAN PATENT APPLICATION

(11) **EP 4 663 238 A2**
(43) Date of publication of application: **17.12.2025**
(21) Application number: 25211950.8
(22) Date of filing: 02.10.2018
(51) Int. Cl.: A61N 5/06

(54) **GLASSES PROVIDED WITH A PHOTON SOURCE, SYSTEM COMPRISING SUCH GLASSES AND USE OF SUCH GLASSES IN PHOTOTHERAPY**

(30) Priority: 03.10.2017 NL 2019663
(62) Divisional of application: 18812330.1
(71) Applicant: Chrono Eyewear B.V., 5026 RM Tilburg (NL)
(72) Inventor: Schoutens, Antonius Maria Cornelis, 5018 AB Tilburg (NL)
(74) Representative: Nederlandsch Octrooibureau

(57) **Abstract**

The invention relates to a glasses provided with a light source for illuminating at least a portion of the eye of a subject wearing the glasses and relates to a system comprising such a glasses. The invention further relates to a system comprising the glasses of the invention and further comprising an external device provided with a processor as well as a computer program which, in use, causes the processor to control the at least one light source in the glasses in accordance with a predetermined program, wherein preferably the desired program is chosen by a wearer of the glasses. The invention also relates to a glasses case. The invention also relates to the glasses of the invention for use in a method of treatment of Parkinson's Disease by phototherapy.

## Description

### TECHNOLOGICAL FIELD OF THE INVENTION

The invention relates to a glasses provided with a light source comprising a photon source for illuminating at least a portion of the eye of a subject wearing the glasses and relates to a system comprising such a glasses. The invention further relates to a system comprising the glasses of the invention and further comprising an external device provided with a processor as well as a computer program which, in use, causes the processor to control the at least one light source in the glasses in accordance with a predetermined program, wherein preferably the desired program is chosen by a wearer of the glasses. The invention also relates to a glasses case. The invention also relates to the glasses of the invention for use in a method of treatment of Parkinson's Disease by phototherapy.

### SUMMARY OF THE INVENTION

A first aspect of the current invention relates to a glasses provided with two transparent spectacle glasses enclosed in a frame, wherein each spectacle glass is positioned in front of an eye of a person by a frame for positioning on the ears and the nose of said person, the glasses further provided with at least one light source integrated in the frame for direct and/or indirect delivery of light substantially from above the spectacle glass to the eyes, wherein a nose frame part of the frame at, at least, a nose frame side portion facing the eye, reflects to the eye at least partially the direct or indirect incident light thereon originating from the light source, wherein the light source comprises a photon source capable of emitting photons with a photon energy of between 2,82 eV and 2,43 eV. Such photon energy of between 2,82 eV and 2,43 eV corresponds to a wavelength of between 440 nm and 510 nm.

In one embodiment, the glasses of the invention comprises at least one light source comprising a photon source, wherein said photon source is capable of emitting photons with a photon energy of between 2,76 eV and 2,48 eV (corresponding to a wavelength of between 450 nm and 500 nm), preferably between 2,67 eV and 2,58 eV (corresponding to a wavelength of between 464 nm and 480 nm), most preferably with a photon energy of about 2,65 eV (corresponding to a wavelength of about 468 nm).

In one embodiment, the glasses of the invention comprises at least one light source comprising a photon source, wherein said photon source is capable of emitting photons with an emission peak wavelength of between 450 nm and 500 nm, preferably between 464 nm and 480 nm, most preferably with an emission peak wavelength of about 468 nm. Preferably, the glasses of the invention comprise two light emitting diodes comprising a photon source capable of emitting photons with an emission peak wavelength of about 468 nm.

In one embodiment, the glasses of the invention comprises at least one light source comprising a photon source, wherein said photon source is capable of emitting photons with a full width at half maximum spectral bandwidth of between 16 nm and 35 nm, preferably between 19 nm and 33 nm, more preferably between 20 nm and 27 nm, most preferably between 24 nm and 26 nm.

In one embodiment, the glasses of the invention comprises at least one light emitting diode comprising the photon source, wherein the photon source has an emission peak wavelength of about 468 nm, wherein the photon source is a p/n semiconductor material consisting of a p-layer and an n-layer, wherein the p-layer and the n-layer are indium-gallium nitride (InGaN), preferably the p-layer is InGaN doped with magnesium and the n-layer is InGaN doped with silicon, wherein the light emitting diode is capable of delivering between 36 lux and 50 lux at the level of an eye of a person wearing the glasses, preferably about 40 lux, and is capable of delivering between 4 lux and 6 lux at the level of the retina of the eye of said person.

In one embodiment, the glasses according to the invention has a frame, wherein said frame above the spectacle glass is provided with at least one light guiding element for indirect delivery of light originating from the at least one light source substantially from above the spectacle glass to the eyes.

In one embodiment, the glasses according to the invention has a frame, wherein said frame above the spectacle glass is provided with at least one light guiding element for indirect delivery of light originating from the at least one light source substantially from above the spectacle glass to the eyes and wherein said light guiding element is provided with an upper side having notches, wherein preferably each notch extends transversely to the longitudinal direction of the light guiding element.

A second aspect of the current invention relates to a system comprising a glasses according to the invention, wherein the system further comprises an external device provided with a processor as well as a computer program which, in use, causes the processor to control the at least one light source in the glasses in accordance with a predetermined program, wherein preferably the desired program is chosen by a wearer of the glasses.

In one embodiment, the glasses of the invention is provided with a frame, said frame being provided with a battery.

A third aspect of the current invention relates to a glasses case for a glasses according to the invention, wherein the battery of the glasses disposed in the glasses case is wirelessly charged by magnetic induction.

A fourth aspect of the invention relates to an applicator provided with at least one light source for delivery of photons to an eye, said light source comprising a photon source capable of emitting photons with a photon energy of between 2,82 eV and 2,43 eV for use in phototherapy, wherein photons emitted by said photon source are delivered on the retina of an eye of a patient subjected to said phototherapy.

A fifth aspect of the invention relates to an applicator provided with at least one light source for delivery of photons to an eye, said light source comprising a photon source capable of emitting photons with a photon energy of between 2,82 eV and 2,43 eV for use in the phototherapy of a neurodegenerative disease, wherein photons emitted by said photon source are delivered on the retina of an eye of a patient subjected to said phototherapy.

A sixth aspect of the invention relates to an applicator provided with at least one light source for delivery of photons to an eye, said light source comprising a photon source capable of emitting photons with a photon energy of between 2,82 eV and 2,43 eV for use in a method of therapy of Parkinson's Disease, wherein photons emitted by said photon source are delivered on the retina of an eye of a patient subjected to said method of therapy.

In one embodiment, the applicator for use according to the invention, is for use in a method of therapy of Parkinson's Disease, wherein by said use at least one of the symptoms of Parkinson's Disease selected from bradykinesia, pain, akinesia, sleep disturbance, depressed mood, sadness, melatonin levels during daytime and dystonia is prevented or treated, or is delayed and/or reduced.

In one embodiment, the applicator for use according to the invention is a glasses according to an embodiment of the invention.

In one embodiment, the applicator for use in the therapy of Parkinson's Disease according to the invention is a glasses according to an embodiment of the invention, wherein the glasses comprises at least one light emitting diode comprising the photon source, wherein the photon source has an emission peak wavelength of about 468 nm, wherein the photon source is a p/n semiconductor material consisting of a p-layer and an n-layer, wherein the p-layer and the n-layer are InGaN, wherein the light emitting diode is capable of delivering between 36 lux and 50 lux at the level of an eye of a treated patient, preferably about 40 lux, and is capable of delivering between 4 lux and 6 lux at the level of the retina of the eye of the treated patient.

### BACKGROUND OF THE INVENTION

WO2014/162271 shows and describes a light therapy device provided with a frame and a number of light sources. The frame comprises two earpieces, a light emitting panel, a light guiding plate and a nose attachment means for positioning the device onto the nose of the user. Figure 6 of WO2014/162271 shows an embodiment of the light guiding plate of the light therapy device. This light guiding plate is roughened locally or coated with white paint in order to reflect the light from the light emitting panel to the eyes of the user. During a light treatment the light guiding plate is not transparent, which means that the user of the light therapy device is not able to look through the glass unimpeded due to the reflected light on the coating and/or on the roughness.

Patent application in the United States of America with number US 2012/0215291 A1 relates to glasses used together with contact lenses to deliver light therapy to the wearer of the glasses. The glasses' lenses or the glasses' frames feature an embedded light source. The lenses or frames of the glasses project light into complimentary contact lenses which refract, diffract or reflect light into the wearer's eyes.

Patent application in the United States of America with number US 2006/0176442 A1 relates to a pair of eyeglasses including a light emitting device. The light emitting device has several light emitting elements positioned around the eyeglasses. The light emitting elements are positioned around the eyeglasses in such way that the pair of eyeglasses projects light into the eyes of the wearer of the pair of eyeglasses from all sides surrounding the eyeglasses.

United States of America patent US 6,857,739 relates to an illuminated eyewear for decorative purposes comprising a light source positioned between the lenses of the eyewear and the bridge of the eyewear. Light emitted by the light source is projected along the edge of the lens and is projected outwardly from the lens in the plain of the major surface of the lens.

### DETAILED DESCRIPTION OF THE INVENTION

It is an aim of the current invention to provide a glasses, preferably a designer glasses, for exposing the eyes to artificial light in a beneficial manner related to energy-consumption and which glasses can be worn continuously without hampering the sight of the wearer during a light treatment.

This aim is achieved with a glasses comprising the aspects as defined in claim 1.

The present invention will be described with respect to particular embodiments and with reference to certain examples but the invention is not limited thereto but only by the claims.

Furthermore, the various embodiments, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a glasses comprising A and B" should not be limited to a glasses consisting only of components A and B, rather with respect to the present invention, the only enumerated components of the glasses are A and B, and further the claim should be interpreted as including equivalents of those components, such as derivatives thereof.

As used herein, the term "may" encompasses the word "can," and the term "may be" encompasses the words "is" or "are," depending on context. Furthermore, presence of the word "may" is intended to explain options for practicing or implementing the disclosure, without limitation.

A first aspect of the current invention relates to a glasses provided with two transparent spectacle glasses enclosed in a frame, wherein each spectacle glass is positioned in front of an eye of a person by a frame for positioning on the ears and the nose of said person, the glasses further provided with at least one light source integrated in the frame for direct and/or indirect delivery of light substantially from above the spectacle glass to the eyes, wherein a nose frame part of the frame at, at least, a nose frame side portion facing the eye, reflects to the eye at least partially the direct or indirect incident light thereon originating from the light source, wherein the light source comprises a photon source capable of emitting photons with a photon energy of between 2,82 eV and 2,43 eV (corresponding to a wavelength of between 440 nm and 510 nm).

In one embodiment, the glasses according to the invention comprises at least one light source wherein the light source comprises a photon source capable of emitting photons with a photon energy of between 2,76 eV and 2,48 eV (corresponding to a wavelength of between 450 nm and 500 nm), preferably between 2,67 eV and 2,58 eV (corresponding to a wavelength of between 464 nm and 480 nm), most preferably with a photon energy of about 2,65 eV (corresponding to a wavelength of about 468 nm).

In one embodiment, the glasses according to the invention comprises at least one light source wherein the light source comprises a photon source capable of emitting photons with an emission peak wavelength of between 450 nm and 500 nm, preferably between 464 nm and 480 nm, most preferably with an emission peak wavelength of about 468 nm.

In one embodiment, the glasses according to the invention comprises at least one light source wherein the light source comprises a photon source capable of emitting photons with a full width at half maximum spectral bandwidth of between 16 nm and 35 nm, preferably between 19 nm and 33 nm, more preferably between 20 nm and 27 nm, most preferably between 24 nm and 26 nm.

In one embodiment, the glasses according to the invention comprises at least one light source wherein the at least one light source is a light emitting diode or an organic light emitting diode or a phosphorescent organic light emitting diode comprising the photon source, preferably a light emitting diode comprising the photon source. A preferred light source according to the invention is a blue-light emitting LED comprising as a photon source a p/n semiconductor material consisting of an InGaN p-layer and an InGaN n-layer.

In one embodiment, the glasses according to the invention comprises at least one light source wherein the at least one light source is a light emitting diode (LED) comprising the photon source.

In one embodiment, the glasses according to the invention comprises at least one light source comprising a photon source, wherein the photon source is a p/n semiconductor material, preferably a p/n semiconductor material consisting of a p-layer and an n-layer.

In one embodiment, the glasses according to the invention comprises at least one light source comprising a photon source, wherein the photon source is a p/n semiconductor material consisting of a p-layer and an n-layer wherein the p-layer and the n-layer are gallium-nitride (GaN) or alloys thereof such as aluminum-gallium nitride (AlGaN) and InGaN, preferably the p-layer and the n-layer are InGaN.

Indium gallium nitride (InGaN, InₓGa₁₋ₓN) is a p/n-semiconductor material made of a mix of gallium nitride (GaN) and indium nitride (InN), known in the art. It is a ternary group III/group V direct bandgap semiconductor.

In one embodiment, the glasses according to the invention comprises at least one light source comprising a photon source, wherein the photon source is a p/n semiconductor material consisting of a p-layer and an n-layer wherein the p-layer and the n-layer are GaN or alloys thereof such as AlGaN and InGaN, preferably the p-layer and the n-layer are InGaN, wherein the p-layer is doped with magnesium and the n- layer is doped with silicon, preferably the p-layer is InGaN doped with magnesium and the n-layer is InGaN doped with silicon.

In one embodiment, the glasses according to the invention comprises at least one light source wherein said at least one light source is capable of delivering between 36 lux and 50 lux at the level of the eye, preferably about 40 lux.

In one embodiment, the glasses according to the invention comprises at least one light source wherein said at least one light source is capable of delivering between 4 lux and 6 lux at the level of the retina of the eye.

In one embodiment, the glasses according to the invention comprises at least one light source wherein said at least one light source is capable of delivering between 8 lux and 10 lux at the level of the eye, and wherein said at least one light source is capable of delivering between 1 lux and 4 lux at the level of the retina of the eye.

Preferably, the glasses of the invention comprises as a light source two to four blue light emitting LEDs, most preferably four of such LEDs, each such a LED having a power of 0,1 Watt. In an embodiment, the glasses of the invention comprises as a light source four blue light emitting LEDs.

In one embodiment, the glasses according to the invention comprises at least one light source wherein said at least one light source further comprises a light emitting diode capable of emitting red light.

In one embodiment, the glasses according to the invention comprises at least one light source wherein said at least one light source further comprises a light emitting diode capable of emitting red light, wherein said light emitting diode capable of emitting red light is capable of emitting light with a wavelength of between 660 nm and 700 nm.

With such a glasses an effective light treatment, or phototherapy, can be executed, wherein the spectacle glasses are transparent continuously, which means that during the execution of a light treatment with the glasses the wearer can look through the spectacle glasses unhindered and the wearer is capable to perform daily routine tasks. With the artificial light emitted by a light source in the glasses it is possible to influence the circadian rhythm of a wearer of the glasses such that for example discomfort of a jetlag and working in shifts/night shifts can be decreased and/or even be prevented. In order for making the layout of the glasses attractive from the viewpoint of design as less components as possible are assembled in the glasses. Therefore, for example the number of light sources is preferably kept to a minimum, for example at maximum four light sources positioned at a distance from each other which are preferably configured as energy-efficient light-emitting diodes.

A minimum number of components in the glasses not only results in a saving of space, though also for a reduction of the energy consumption of the glasses. Herewith the volume and mass of the battery can be kept minimally, which contributes in an enhanced manner to a more compact glasses for the execution of artificial-light treatment. By providing a compact glasses the glasses appears as a regular glasses without light source with regard to design. Herewith the glasses can be worn continuously in the public without standing out that a light treatment can be executed with the glasses. Furthermore the light treatment can be started and terminated automatically at a predetermined time-point without the necessity for the wearer of the glasses to perform an act. In order to guarantee the effective performance of the artificial light in the glasses without being at the expense of the vision through the transparent spectacle glasses or an increased energy consumption, the light originating from the light source is delivered to the eyes directly or indirectly from above, which means that the light is delivered above the spectacle glasses to the eye of the subject wearing the glasses. A wearer of the glasses will experience no hindrance from the artificial light delivered from above to the eyes during a light treatment. The light source will for the purpose be integrated in the portions of the frame positioned above the eyes or in the frame legs, wherein in case of a light source in a frame leg the artificial light is guided to the portions of the frame positioned above the eyes by light guides and subsequently delivered to the eyes in an indirect manner. The delivery of light to the eyes in an indirect manner provides more comfort to the user of the glasses than when the light is guided to the eyes directly via the light source.

In order to optimize in the glasses in an energy-beneficial manner the amount of artificial light to the eyes, the lower part of the frame at least comprising the nose frame part is configured such that a nose frame side portion which is at least facing the eye of the wearer reflects at least in part the directly or indirectly incident light originating from the light source. This reflection of the nose frame side portion which is facing the eye of the wearer is at least 50%, preferably more than 85%. This way, artificial light that is lost otherwise during a light treatment is yet reflected to the eyes of the user.

An additional optional feature for the frame of the glasses is that at least portions of the surface of the nose frame side portion which is facing the eye of the wearer reflect the light diffusely. This way the comfort for a user can be increased during a light treatment. Another optional feature is to configure at least portions of the nose frame side portion which is facing the eye of the wearer curved to direct the reflected light specifically to the eye or to a desired location in the eye in order to enhance the effect of the artificial light such that the duration of the light treatment can be shortened, allowing a decrease in the energy consumption of the glasses.

With such a configured glasses a maximum result is achieved in an energy beneficial way with the artificial light emitted by a light source. Lowering the number of components and the total energy consumption of the glasses when in use results in an increased freedom of design for the glasses or sunglasses for executing a treatment with artificial light, such that with regard to the appearance it is possible to establish an attractive designer glasses for the execution of a light treatment.

For example, by using the glasses with artificial light treatment during winter day with relatively short periods of day light, it is possible for the wearer of the glasses' body to artificially lengthen the period of day light during winter, such that the wearer's condition can be improved with the glasses or the energy level of the wearer can be enlarged. In addition, the glasses can be used to optimize the moment at which a performance has to be delivered. For an athlete it is for example not unthinkable that the performance to be delivered takes place for commercial reasons at a time of the day that is less beneficial for the body of the athlete such as for example late in the evening. By using the glasses with the light source therein blue light can be emitted to the eyes of the wearer whereby the production of melatonin is inhibited and the production of cortisol is stimulated at a moment at which presumably the reverse natural effect would occur without glasses. This way, the day-night effects on a human being that regularly hinder a top performance are minimized.

In one embodiment, the glasses of the invention comprises at least one light source comprising a photon source for emitting photons with blue wavelength, wherein said light source is capable of delivering about 40 lux at the level of the lens of the eye, according to the invention. This way, the delivery of the 40 lux by the glasses of the invention results in optimal suppression of melatonin levels in the wearer of the glasses, according to the invention.

Preferably, the emission peak wavelength of the photon source comprised by the at least one light source in the glasses of the invention is about 468 nm, according to the invention. It is part of the invention that suppression of melatonin production/release in the body of the wearer of glasses of the invention is efficient and optimal when the glasses comprise at least one light source comprising a photon source capable of delivering about 40 lux at the level of the lens of the eye, wherein the photon source emits photons having an emission peak wavelength of about 468 nm, according to the invention.

Preferably, the glasses of the invention provides for a photon source capable of delivering about 36 lux to 50 lux, preferably about 40 lux, at the level of the lens of the eye, and capable of delivering between about 4 lux and 6 lux at the level of the retina of the eye, according to the invention.

The spectacle glasses can be made of glass or plastic. The spectacle glasses can be connected with each other as one piece.

The use of whitening agents, also referred to as optical brighteners, can enhance the percentage of reflectance of visible light even to above 100 percent. The whitening agents converse invisible ultraviolet light that is harmful to the eye to visible blue light, such that the perceived reflection is higher than the initial amount of light originating from the light source. The whitening agent establishes that radiation is added to the reflection of the visible light.

For simulating a dark environment the glasses can be provided with filter lenses that filter the blue spectrum of the (day) light and the UV light. Transparent red spectacle glasses are for example suitable for simulating a dark environment. This way, the biological production of the hormone melatonin is not suppressed, such that the body receives an endogenous signal to reduce the day time activities and to prepare for the night.

For delivery of light originating from a light source in an indirect way to the eyes a light guiding element can be used.

In one embodiment, the glasses according to the invention has a frame, wherein said frame above the spectacle glass is provided with at least one light guiding element for indirect delivery of light originating from the at least one light source substantially from above the spectacle glass to the eyes.

In one embodiment, the glasses according to the invention has a frame, wherein said frame above the spectacle glass is provided with at least one light guiding element for indirect delivery of light originating from the at least one light source substantially from above the spectacle glass to the eyes and wherein said light guiding element is provided with an upper side having notches, wherein preferably each notch extends transversely to the longitudinal direction of the light guiding element.

In one embodiment, the glasses according to the invention has a frame, wherein said frame above the spectacle glass is provided with at least one light guiding element for indirect delivery of light originating from the at least one light source substantially from above the spectacle glass to the eyes and wherein said light guiding element is provided with an upper side having notches, wherein preferably each notch extends transversely to the longitudinal direction of the light guiding element, wherein the notches in the direction of the nose frame part become deeper.

In one embodiment, said light guiding element is provided with an upper side having notches, wherein the notches in the direction of the nose frame part become evenly deeper. In one embodiment, said light guiding element is provided with an upper side having notches, wherein the distances between centers of the notches are constant.

By applying a light guiding element in the glasses the glasses becomes more user friendly during a light treatment because this way direct emission of light originating from the light source is minimized. The light guiding element can be integrated in the frame. The light guiding element comprises notches in an upper surface thereof for reflecting the light originating from the light source when in use to a desired position. When the glasses are worn the light guiding element is located above the eyes. The notches preferably extend transversely to a longitudinal direction of the elongated light guiding element, wherein the notches in the direction of the nose frame part deepen uniformly for reflecting the light originating from the light source to the desired position in the eye. The notches have a triangular cross-section wherein the distance between the centers of the notches is almost constant. This ensures an even distribution of light.

In one embodiment, the glasses of the invention comprises a light guiding element which is a semi-transparent element and which has a predominantly dark-colored, e.g. black-colored, surface at the side of the light guiding element facing the eye from above the eye, with at least one transparent portion in said surface for passing of light such that light is delivered through said transparent portion of the light guiding element from preselected discrete one or more position(s) above the eye of the wearer of the glasses to said eye of the wearer of the glasses of the invention, according to the invention.

In an alternative embodiment, the glasses of the invention comprises a light guiding element which is completely transparent for the light to be guided, such that light is delivered through said transparent light guiding element from above the eye of the wearer of the glasses to said eye of the wearer of the glasses of the invention, according to the invention.

In one embodiment, the glasses of the invention is either provided with a semi-transparent light guiding element or provided with a completely transparent light guiding element, wherein the light guiding element is further provided with an upper side having notches, wherein preferably each notch extends transversely to the longitudinal direction of the light guiding element. Preferably, said notches in the direction of the nose frame part become deeper, preferably the notches in the direction of the nose frame part become evenly deeper, and preferably, the notches in the uppers side of the light guiding element are positioned at constant distances from each other.

The frame of the glasses can be provided with a support frame for supporting the spectacle glasses. The support frame comprises a support-frame upper portion which is positioned above the eyes when the glasses are worn correctly, and which extends between the frame legs of the glasses. The center of the support-frame upper portion can be connected to the nose frame part. The support-frame upper portion of the glasses can be provided with the above mentioned light guiding element in order to indirectly guide the artificial light from above from the at least one light source to the eyes. Moreover, indirect emission allows for more freedom in design since the position of the light source is optimizable. The light source is for example positioned further away from the eye such that the eye experiences no or just minimal hinder from the warmth produced by the light source.

The support frame of the frame encloses at least half of the perimeter of the spectacle glasses wherein at least the portion of the support frame at eye level when the glasses are worn and/or the portion of the support frame that is located lower than the eye when the glasses are worn reflects the directly or indirectly emitted light from the light source at the side of the support frame that is facing the eye of the wearer. The reflection is at least 50%, preferably more than 85%.

A second aspect of the current invention relates to a system comprising a glasses according to the invention, wherein the system further comprises an external device provided with a processor as well as a computer program which, in use, causes the processor to control the at least one light source in the glasses in accordance with a predetermined program, wherein preferably the desired program is chosen by a wearer of the glasses.

The invention also relates to a system comprising the glasses described in this document with a communication unit and an external device provided with a processor, and in addition a computer program which, when in use, effectuates the processor to control the at least one light source in the glasses according to a predetermined program, wherein preferably the desired program can be selected by the wearer of the glasses. The external device is preferably a portable apparatus such as a smartphone / tablet or the like, wherein the computer program can be an app that is installable on the telephone which app can communicate wirelessly with the glasses via Bluetooth technology, for example to periodically start or to end a light treatment according to a predetermined or selected program.

A third aspect of the current invention relates to a glasses case for a glasses according to the invention, wherein the battery of the glasses disposed in the glasses case is wirelessly charged by magnetic induction.

The invention also relates to a glasses case for a glasses described in this application, wherein the glasses case and the glasses are provided with for example Qi technology such that the battery of the glasses placed in the glasses case is wirelessly chargeable by magnetic induction. Preferably, the glasses case comprises its own battery such that the portable glasses case can be used for charging the glasses without cables.

The battery that is comprised by the glasses of the invention is for example a rechargeable lithium ion polymer battery.

A fourth aspect of the invention relates to an applicator provided with at least one light source for delivery of photons to an eye, said light source comprising a photon source capable of emitting photons with a photon energy of between 2,82 eV and 2,43 eV for use in phototherapy, wherein photons emitted by said photon source are delivered on the retina of an eye of a patient subjected to said phototherapy.

The term "applicator" has its normal scientific meaning, and here refers to a carrier device for a light source comprising a photon source, such that photons emitted by the photon source comprised by the light source are delivered to the lens of the eye of a host and are delivered to the retina of the eye of the host, when the photon source is applied to, or administered to, said host by way of bringing the applicator in close proximity to the eye of the host. According to the invention, a glasses of the invention is such an applicator, *i.e.* a carrier device for a light source comprising a photon source, such that photons emitted by the photon source comprised by the light source are delivered directly or indirectly to the lens of the eye of a host wearing the glasses and are delivered to the retina of the eye of the host wearing the glasses, when the photon source is applied to, or administered to, said host by way of bringing the applicator in close proximity to the eye of the host, *i.e.* by way of wearing the glasses by the host.

An important benefit of the glasses of the invention for use in the therapy of Parkinson's Disease, is that the dosage of photons emitted by the photon source comprised by the light source in said glasses, is highly consistent and stable, from administration to administration throughout time of therapy. The glasses are worn such that the photon source is delivering the photons at the desired photon energy (emission peak wavelength of 468 nm) from a constant distance between the applicator, here the part of the glasses positioned above the eye of the patient, and the retina of the eyes of the patient. Dosing is conveniently adjusted either by altering the dosing frequency for example over the day or throughout the week, or by adjusting the duration of the delivery of photons by the photon source of the glasses on the retina, or by both altering the dosing frequency and the illumination time, according to the invention. Furthermore, due to the design of the applicator, here the glasses of the invention, a dose of blue-light photons can be administered to a Parkinson's Disease patient by administering the photon source to the patient upon letting the patient wear a glasses of the invention. Moreover, the phototherapy of Parkinson's Disease by using the glasses of the invention is easy and straightforward, such that a PD patient is able to administer the photon source at any moment of the day or night by him/herself, without the need to visit or see a practitioner, *etc.* In addition, by the aid of the application ('app'), for example a practitioner is able to pre-program the photon dosing, photon dosing frequency, duration of an administration of photons by the photon source of the glasses of the invention; the PD patient conveniently can wear the glasses during daytime and thereby continuously administer the photon source to the close proximity of the eyes such that photons can be delivered virtually unnoticed by the patient, according to the preprogrammed treatment plan.

The inventors surprisingly found that administering a photon source to the eyes of a host with the aid of an applicator, e.g. the glasses of the invention, that is to say, by bringing in close proximity to the eyes of a host a light source comprising said photon source with the aid of an applicator, beneficial effects are obtained with regard to the relief of symptoms related to neurodegenerative disease, e.g. Parkinson's Disease, related to the emission and subsequent delivery of photons by the photon source directly or indirectly on the lens of the eye of a patient suffering from said disease, and on the retina of said patient.

A fifth aspect of the invention relates to an applicator provided with at least one light source for delivery of photons to an eye, said light source comprising a photon source capable of emitting photons with a photon energy of between 2,82 eV and 2,43 eV for use in the phototherapy of a neurodegenerative disease, wherein photons emitted by said photon source are delivered on the retina of an eye of a patient subjected to said therapy.

A sixth aspect of the invention relates to an applicator provided with at least one light source for delivery of photons to an eye, said light source comprising a photon source capable of emitting photons with a photon energy of between 2,82 eV and 2,43 eV for use in a method of therapy of Parkinson's Disease, wherein photons emitted by said photon source are delivered on the retina of an eye of a patient subjected to said method of therapy.

Parkinson's Disease (PD) is a neurodegenerative disease and belongs to a group of conditions referred to as motor system disorders and PD involves the malfunction and death of neurons in an area of the brain called the substantia nigra and these neurons are the vital nerve cells in the brain. Some of these dying neurons produce dopamine which is a chemical that sends messages to the part of the brain that controls movement and coordination.

With the progression of PD, the amount of dopamine produced in the brain decreases, leaving a person unable to control movement normally. It usually affects people over the age of 60. PD is both chronic and progressive. Although some people become severely disabled, others experience only minor motor disruptions.

Early symptoms of PD are subtle and occur gradually. The four primary symptoms of PD are:
- tremor, or trembling in hands, arms, legs, jaw, and face;
- rigidity, or stiffness of the limbs and trunk;
- bradykinesia, or slowness of movement; and
- postural instability, or impaired balance and coordination.

Tremor is the major symptom for some human PD patients, while for other patients tremor is only a minor complaint and other symptoms are more troublesome. It is currently not possible to predict which symptoms will affect an individual PD patient, and the intensity of the symptoms also varies from patient to patient. In some human PD patients the disease progresses more quickly than in other patients.

At present, there is no cure for PD, but a variety of medications provide relief from the symptoms of PD. Usually, affected individuals are given levodopa combined with carbidopa. Carbidopa delays the conversion of levodopa into dopamine until it reaches the brain. Nerve cells can use levodopa to make dopamine and replenish the brain's dwindling supply. Although levodopa helps at least three-quarters of parkinsonian cases, not all symptoms respond equally to the drug. Bradykinesia and rigidity are symptoms that respond best, while tremor may be only marginally reduced. Problems with balance and other symptoms may not be alleviated at all. Anticholinergics may help control tremor and rigidity. Other drugs, such as bromocriptine, pramipexole, and ropinirole, mimic the role of dopamine in the brain, causing the neurons to react as they would to dopamine. An antiviral drug, amantadine, also appears to reduce symptoms. In May 2006, the FDA approved rasagiline to be used along with levodopa for patients with advanced PD or as a single-drug treatment for early PD. In March 2017, the FDA approved safinamide tablets as an add-on treatment for individuals with PD who are currently taking levodopa/carbidopa and experiencing "off" episodes (when the person's medications are not working well, causing an increase in PD symptoms). For some human PD patients, a treatment called deep brain stimulation (DBS) may also be used. For this treatment, a surgeon places wires in the patient's brain. The wires carry tiny electrical signals to the parts of the brain that control movement. These little signals can help those parts of the brain work better. DBS can reduce the need for levodopa and related drugs, which in turn decreases the involuntary movements called dyskinesias that are a common side effect of levodopa. It also helps to alleviate fluctuations of symptoms and to reduce tremors, slowness of movements, and gait problems. DBS requires careful programming of the stimulator device in order to work correctly.

Application of light therapy in PD is tested in the field. Symptomatic benefits could perhaps occur in PD patients by using red to infrared light therapy (wavelength is between 600 nm and 1,070 nm) to treat PD patients, based on the results of tests with animals. Administering photons with such photon energy of 2,066 eV to 1,159 eV (yellow light to red light to infrared radiation) to the retina of the eyes of a patient suffering from PD could perhaps be helpful because PD patients commonly suffer from excessive daytime sleepiness, fatigue, sleep disorders, as well as depression.

In one embodiment, the applicator for use according to the invention, is used for the therapy of Parkinson's Disease wherein by said use at least one of the symptoms of Parkinson's Disease selected from bradykinesia, pain, akinesia, sleep disturbance, depressed mood, sadness, melatonin levels during daytime and dystonia is prevented or treated, or is delayed and/or reduced, according to the invention. In particular, by application of the glasses of the invention as an applicator for administering a photon source capable of emitting about 40 lux at the level of the lens of the eye and about 4 lux to 6 lux on the retina of the eye, wherein the emitted photons have an emission wavelength maximum of about 468 nm by using four blue-light emitting LED light sources comprising InGaN p/n-semiconductor material, melatonin levels decreased during daytime, resulting in reduction of sleepiness during the day, sleep disturbances were treated to a great extent in PD patients, resulting in an earlier onset of sleeping, patients suffered from less and shorter awakenings during night time, patients experienced beneficial and desirable earlier awakening in the morning, patient were able to sleep longer periods of time, suffered less from pain, even benefited from disappearance of pain, experienced from decreased muscle rigidity, reduced dystonia, made movement of limbs less cumbersome, according to the invention.

Patients suffering from Parkinson's Disease were subjected to phototherapy by administration of the photon source comprised by the glasses of the invention as the carrier applicator, twice daily for about 30 minutes to 1 hour of delivery of photons by the photon source to the retina of the eyes of the patients. The first dosage of photons of the day was delivered to the retina of the eyes of the patients by administering the photon source by wearing the glasses of the invention, at a time point in the morning, typically between 0 hours and 3 hours after awakening in the morning after a night of sleep. The second dosage of photons of the day was delivered to the retina of the eyes of the patients by administering the photon source by wearing the glasses of the invention, at a time point during the day, typically between 10 hours and 14 hours after the first administration of the photon source and emission of photons therewith. For example, PD patients are subjected to phototherapy using the glasses of the invention, by administering the photon source in the morning between half past seven and ten o'clock, for example at eight o'clock, and in the evening, for example between ten o'clock and eleven o'clock. Typically, PD patients to whom the photon source comprised by the glasses of the invention was administered for twice daily delivery of photons on the retina of the eyes, could lower the daily dose of dopamine replacement drug required to suppress sleep disturbances during the night, with improved quality of sleep during the night, preferably the dependency on dopamine replacement drug was not apparent anymore after using the glasses of the invention in phototherapy according to the invention.

Without wishing to be bound by theory, it is thought that the observed beneficial effects of the administration of a photon source emitting blue light at the retina of a host, in particular the administration of a photon source capable of emitting photons with an emission peak wavelength of about 468 nm, with regard to the relief of disease symptoms, *i.e.* symptoms of PD, is the consequence of the activation of either photoreceptor rhodopsin comprising 11-cis retinal present in rods of the retina, or photoreceptor melanopsin comprising 11-cis retinal, or S-receptor present in cones of the retina, or any combination thereof. According to the invention, providing about 40 lux at the level of the lens of the eye and providing about 4 lux to 6 lux at the level of the retina of the eye is beneficial and enough to cause the relief of PD symptoms in a patient suffering from PD. The frequency of administration of the photon source as well as the duration of the exposure of the retina to photons emitted by said photon source are optimally established on a patient case by case basis. Typically, PD patients benefit from administration of a photon source capable of emitting photons with an emission peak wavelength of 468 nm, and subsequent delivery of 4 lux to 6 lux at the level of the retina for about 30 minutes twice a day, wherein the photon source is administered by the use of a glasses of the invention as applicator carrying the photon source comprised by the light source of said glasses.

In one embodiment, the applicator for use according to the invention, is used for phototherapy such as used for the therapy of Parkinson's Disease wherein the applicator is a glasses according to a glasses of the invention and exemplified by a glasses of any one of the embodiments of the invention or by a system according to the invention comprising such a glasses of any one of the embodiments of the invention.

In one embodiment, the applicator for use according to the invention is a glasses of the invention for use in phototherapy, such as for use in the therapy of Parkinson's Disease, wherein the glasses comprises at least one light emitting diode comprising the photon source, wherein the photon source has an emission peak wavelength of about 468 nm, wherein the photon source is a p/n semiconductor material consisting of a p-layer and an n-layer, wherein the p-layer is InGaN and the n-layer is InGaN, wherein the light emitting diode is capable of delivering between 36 lux and 50 lux at the level of an eye of a treated patient, preferably about 40 lux, and is capable of delivering between 4 lux and 6 lux at the level of the retina of the eye of the treated patient.

A further aspect of the invention relates to an applicator for use in phototherapy, wherein the applicator is provided with at least one light source for delivery of photons to an eye, said light source comprising a photon source capable of emitting photons with a photon energy of between 2,82 eV and 2,43 eV, wherein photons emitted by said photon source are delivered on the retina of an eye of a patient subjected to said phototherapy, wherein the applicator is a glasses of the invention such as a glasses of any of the detailed embodiments, or is a system of the invention.

A further aspect of the invention relates to an applicator for use as a medicament in phototherapy, wherein the applicator is provided with at least one light source for delivery of photons to an eye, said light source comprising a photon source capable of emitting photons with a photon energy of between 2,82 eV and 2,43 eV, wherein photons emitted by said photon source are delivered on the retina of an eye of a patient subjected to said phototherapy, wherein the applicator is a glasses of the invention such as a glasses of any of the detailed embodiments, or is a system of the invention.

A further aspect of the invention relates to an applicator for use in phototherapy of a neurodegenerative disease, wherein the applicator is provided with at least one light source for delivery of photons to an eye, said light source comprising a photon source capable of emitting photons with a photon energy of between 2,82 eV and 2,43 eV, wherein photons emitted by said photon source are delivered on the retina of an eye of a patient subjected to said therapy, wherein the applicator is a glasses of the invention such as a glasses of any of the detailed embodiments, or is a system of the invention.

A further aspect of the invention is an applicator for use in a method for the therapy of Parkinson's Disease, wherein the applicator is provided with at least one light source for delivery of photons to an eye, said light source comprising a photon source capable of emitting photons with a photon energy of between 2,82 eV and 2,43 eV, wherein photons emitted by said photon source are delivered on the retina of an eye of a Parkinson's Disease patient subjected to said method of therapy, wherein the applicator is a glasses of the invention such as a glasses of any of the detailed embodiments, or is a system of the invention.

A further aspect of the invention is an applicator for use in a method of therapy of Parkinson's Disease, wherein the applicator is provided with at least one light source for delivery of photons to an eye, said light source comprising a photon source capable of emitting photons with a photon energy of between 2,82 eV and 2,43 eV, wherein photons emitted by said photon source are delivered on the retina of an eye of a Parkinson's Disease patient subjected to said method of therapy, wherein the applicator is a glasses of the invention such as a glasses of any of the detailed embodiments, or is a system of the invention.

Preferred is the applicator for use in a method for the therapy of Parkinson's Disease according to the invention, wherein by said use at least one of the symptoms of Parkinson's Disease selected from bradykinesia, pain, akinesia, sleep disturbance, depressed mood, sadness, melatonin levels during daytime and dystonia is prevented or treated, or is delayed and/or reduced.

Also preferred is the applicator for use in a method of therapy of Parkinson's Disease according to the invention, wherein by said use at least one of the symptoms of Parkinson's Disease selected from bradykinesia, pain, akinesia, sleep disturbance, depressed mood, sadness, melatonin levels during daytime and dystonia is prevented or treated, or is delayed and/or reduced.

Typically, the applicator for use in the phototherapy according to the invention or the applicator for use in the method for the therapy of Parkinson's Disease according to the invention is an applicator of the invention, wherein the glasses comprises at least one light emitting diode comprising the photon source, wherein the photon source has an emission peak wavelength of about 468 nm, wherein the photon source is a p/n semiconductor material consisting of a p-layer and an n-layer, wherein the p-layer is InGaN and the n-layer is InGaN, wherein the light emitting diode is capable of delivering between 36 lux and 50 lux at the level of an eye of a treated patient, preferably about 40 lux, and is capable of delivering between 4 lux and 6 lux at the level of the retina of the eye of the treated patient.

Typically, the applicator for use in the method of therapy of Parkinson's Disease according to the invention is an applicator of the invention, wherein the glasses comprises at least one light emitting diode comprising the photon source, wherein the photon source has an emission peak wavelength of about 468 nm, wherein the photon source is a p/n semiconductor material consisting of a p-layer and an n-layer, wherein the p-layer is InGaN and the n-layer is InGaN, wherein the light emitting diode is capable of delivering between 36 lux and 50 lux at the level of an eye of a treated patient, preferably about 40 lux, and is capable of delivering between 4 lux and 6 lux at the level of the retina of the eye of the treated patient.

In one embodiment, the applicator for use according to the invention is a glasses of the invention for use in phototherapy, such as for use in the therapy of Parkinson's Disease, wherein the photons emitted by the photon source comprised by said glasses are delivered at the retina of a patient at least once daily for between about 15 minutes and 60 minutes, preferably for about 30 minutes.

In one embodiment, the applicator for use according to the invention is a glasses of the invention for use in phototherapy, such as for use in the therapy of Parkinson's Disease, wherein the photons emitted by the photon source comprised by said glasses are delivered at the retina of a patient once daily for about 30 minutes, the delivery of the photons starting after between about 0 minutes and four hours of the time of awakening after overnight sleep, preferably between 30 minutes and 2 hours.

In one embodiment, the applicator for use according to the invention is a glasses of the invention for use in phototherapy, such as for use in the therapy of Parkinson's Disease, wherein the patient is treated for a time period of at least one week, preferably at least one month, more preferably at least one year, most preferably for the rest of the lifespan of the patient.

One aspect of the invention relates to the use of the glasses of the invention for treating a host suffering from Parkinson's Disease, wherein said glasses comprises at least one light emitting diode comprising a photon source, wherein said photon source has an emission peak wavelength of about 468 nm, wherein the photon source is a p/n semiconductor material consisting of a p-layer and an n-layer, wherein the p-layer and the n-layer are InGaN, and wherein the light emitting diode is capable of delivering between 36 lux and 50 lux at the level of an eye of a treated patient, preferably about 40 lux, and is capable of delivering between 4 lux and 6 lux at the level of the retina of the eye of the treated patient. Preferably, the host is a human subject suffering from Parkinson's Disease.

One aspect of the current invention relates to a method of treating a host suffering from Parkinson's Disease, the method comprising administering to the host a photon source such that photons originating from the photon source are delivered at the eye and at the level of the retina of the eye of the treated host, said photon source being a p/n semiconductor material consisting of a p-layer and an n-layer, wherein the p-layer and the n-layer are InGaN, wherein said photon source has an emission peak wavelength of about 468 nm and wherein the method of treatment comprises the delivery of between 36 lux and 50 lux at the level of an eye of a treated host, preferably about 40 lux, and further comprises the delivery of between 4 lux and 6 lux at the level of the retina of the eye of the treated host, and wherein the photon source is an integral part of a glasses of the invention provided with at least one light source comprising said photon source for delivery of photons to the lens of an eye of the host and to the retina of an eye of said host.

Other additional aspects of the glasses are described in the dependent claims and further exemplifying embodiments according to the invention are described below, which further embodiments should not be interpreted as limiting the present invention in any way.

### BRIEF DESCRIPTION OF THE DRAWINGS

Figure 1 shows a front perspective view of a glasses;
Figure 2 shows a rear perspective view of a glasses;
Figure 3 shows an exploded rear view of the glasses shown in Figure 1 and 2;
Figure 4 shows an exploded front view of the glasses shown in Figure 1 and 2;
Figure 5 shows an exploded side view of the glasses shown in Figure 1 and 2;
Figures 6a and 6b show a light guiding element for another embodiment of the glasses;
Figure 7 shows a perspective rear view of a glasses; and
Figure 8 shows an embodiment of a nose frame part and light guiding elements being a single part.

In the figures, corresponding parts have the same reference number.

### EXEMPLIFYING EMBODIMENTS OF THE INVENTION

The glasses is now explained by an embodiment as shown in the incorporated figures.

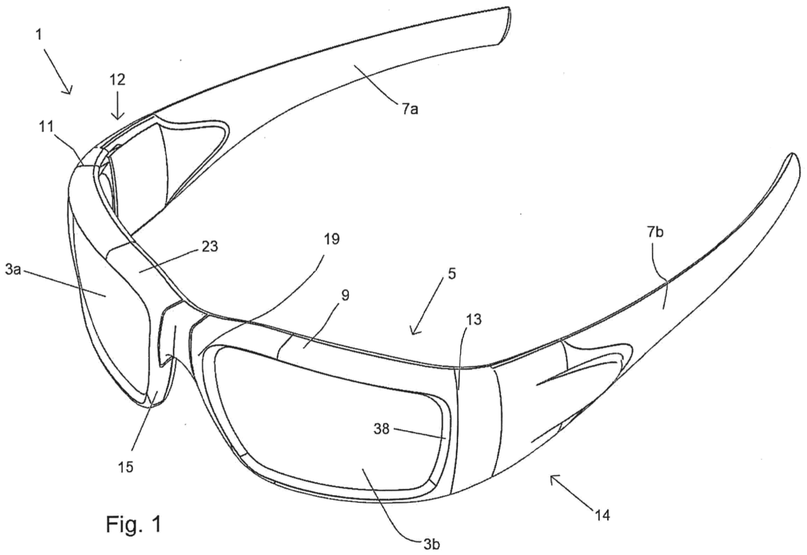

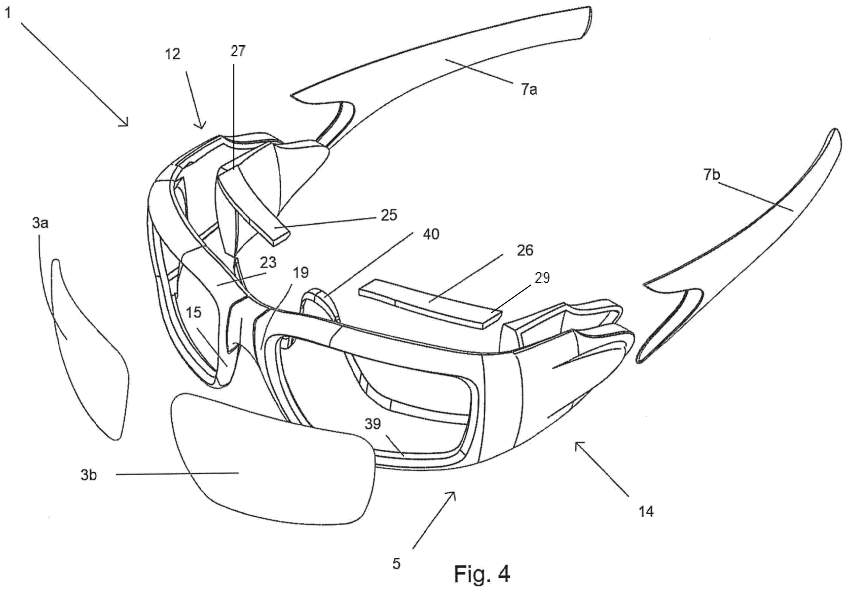

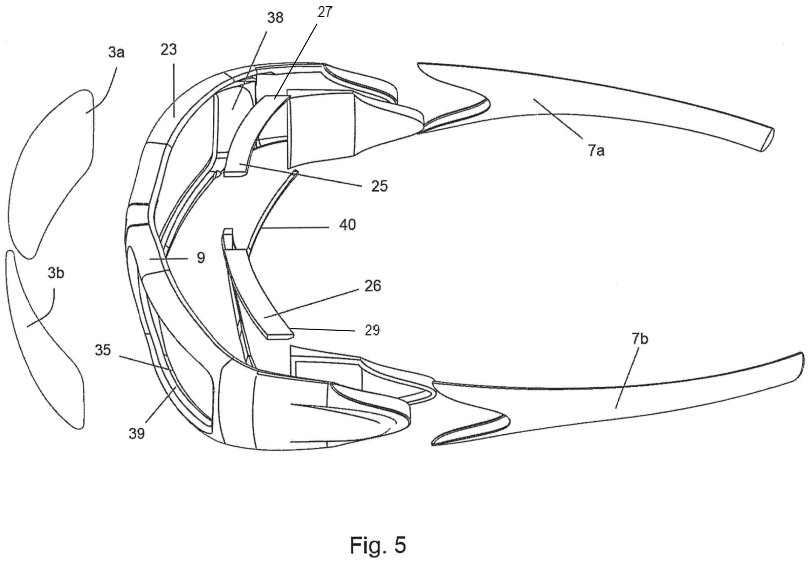

The figures 1-5 show a glasses 1 comprising two transparent spectacle glasses 3a, 3b contained by a frame 5. The spectacle glasses 3a, 3b comprise the shape of conventional spectacle glasses. The spectacle glasses 3a, 3b can be made as colored spectacle glasses. Optionally, the spectacle glasses are filter lenses for filtering of blue light and/or UV light, or the spectacle glasses are filter lenses for filtering of red light. The spectacle glasses can be releasably connected to the frame 5, such that the user can exchange the filter lenses for filtering blue light and/or UV light for filter lenses for filtering red light, or, when desired, for conventional spectacle glasses.

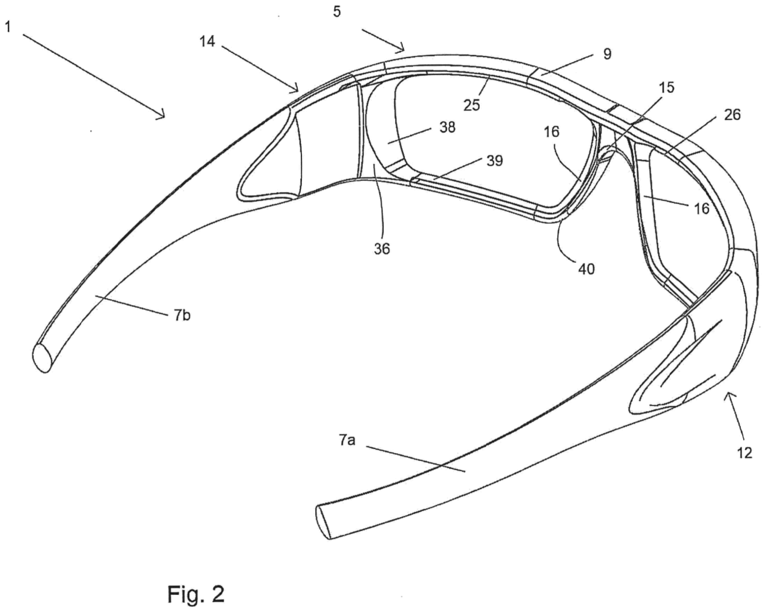

The frame 5 further comprises two frame legs 7a, 7b. In the figures 1 and 2 the glasses 1 is shown in use position in which the glasses 1 can be worn by an individual. Each spectacle glass 3a, 3b is positioned in front of one of the two eyes of an individual by frame 5 positioned at the ears and the nose of said individual. The glasses shown in the figures can be provided as sun glasses with spectacle glasses 3a, 3b for sun glasses.

The frame 5 further comprises a support frame 9 for supporting spectacle glasses 3a, 3b. The support frame 9 is connected at a first extremity 11 (left extremity in Figure 1) with a first frame side part 12 and connected at a second extremity 13 (right extremity in Figure 1) with a second frame side part 14. The frame side parts 12, 14 are connected to frame legs 7a, 7b at an extremity distal from support frame 9.

The glasses 1 is provided with two light sources (not shown), wherein the first light source in the first frame side part 12 is incorporated into an extremity thereof located near the spectacle glass 3a, whereas the second light source in the second frame side part 14 is incorporated into an extremity thereof located near the spectacle glass 3b.

The light sources are light-emitting diodes (LEDs), which LEDs can emit red and/or blue light. The blue light has a wavelength of 480-500 nanometer or a wavelength of 440-510 nm, preferably between 450 nm and 500 nm, more preferably between 464 nm and 480 nm, most preferably, the blue light has an emission peak wavelength of about 468 nm. The red light has a wavelength of 660-700 nanometer. It is possible to provide the glasses 1 with blue-light emitting LEDs, only. With the LEDs a light treatment can be performed with the glasses 1, for inhibiting or stimulating the wearer's production of melatonin / cortisol. In one embodiment, the glasses of the invention comprises at least one light source comprising at least four blue-light emitting LEDs. In an alternative embodiment, the glasses of the invention comprises at least one light source comprising two blue-light emitting LEDs. Said two blue-light emitting LEDs preferably have an emission peak wavelength of 468 nm, and preferably, with the glasses of the invention comprising said two blue-light emitting LEDs about 40 lux can be delivered at the level of the eye of the wearer of the glasses, when the photon source of said LEDs is emitting photons, and preferably about 4 lux to 6 lux can be delivered at the level of the retina of said wearer of the glasses.

The support frame 9 comprises a nose frame part 15. The nose frame part 15 is that portion of the frame 5 which is located around the nose when the glasses 1 is worn by a user. The nose frame part 15 comprises a nose contact surface, a nose frame front side portion 19 directed away from the wearer, an inner surface 16 for supporting a part of the spectacle glass as well as a nose frame side portion 17 facing the eye when the glasses 1 is worn. In the presented glasses 1 the nose contact surface and the nose frame front side portion 19 seamlessly form one piece. The support frame 9 comprises also a support frame upper part 23 which is positioned above the eyes of the wearer when the glasses 1 is worn correctly, and which extents in between frame legs 7a, 7b of the glasses 1. At its center, the support frame upper part 23 is connected with the nose frame part 15.

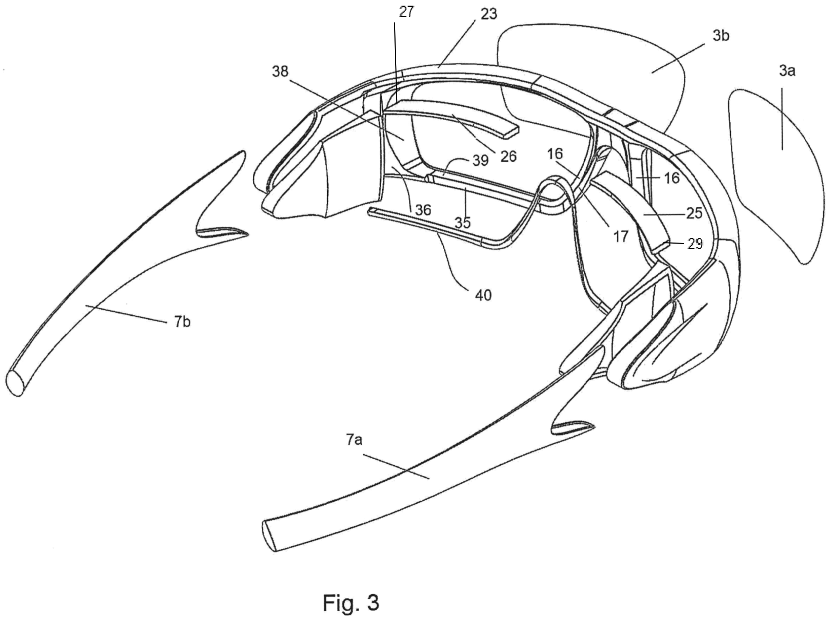

As shown in Figures 3-5, the support frame upper part 23 of the glasses 1 is provided with two plate-shaped elongated light guiding elements 25, 26 integrated therein. The light emitted by the LEDs is guided indirectly to the eyes with the light guiding elements 25, 26. An extremity 27, 29 of each light guiding element 25, 26, that is directed away from the nose frame part 15, is in connection with the LEDs in the frame side parts 12, 14 such that the artificial light of the LEDs is emitted indirectly to the eyes from above via the plate-shaped light guiding elements 25, 26 integrated in the support frame upper part 23. It is possible that in the glasses 1 no artificial light is emitted directly from a LED to the eyes of a user, such that the light treatment executed with the glasses 1 is only using indirect light. This way, for the wearer the comfort of the glasses 1 during a light treatment is enhanced. In one embodiment, the surface of the plate-shaped elongated light guiding element that faces downwardly, when the glasses are worn, is only transparent for photons at one or more discrete portions of said surface. This way, photons delivered by the photon source comprised by the light source in the glasses of the invention, can be directed to pre-determined portions of the eye and to predetermined portions of the retina of the eye of the wearer of the glasses. In an alternative embodiment, the complete surface of the plate-shaped elongated light guiding element that faces downwardly, when the glasses are worn, is transparent for photons. This way, the photons emitted by the photon source comprised by the light source in the glasses of the invention are delivered efficiently to the eye and the retina of the wearer of the glasses over the whole length of the light guiding element located above the eye of the wearer of the glasses, with less loss of energy (photons) when compared to a partially non-transparent light guiding element.

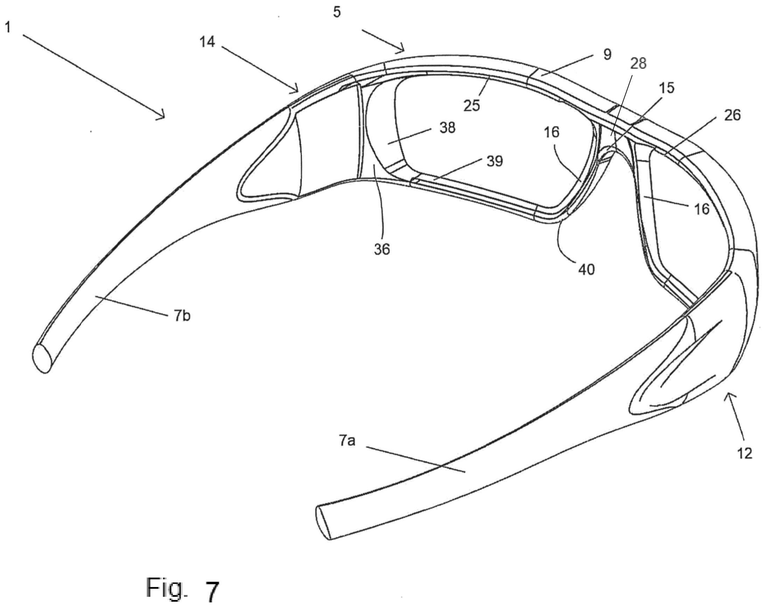

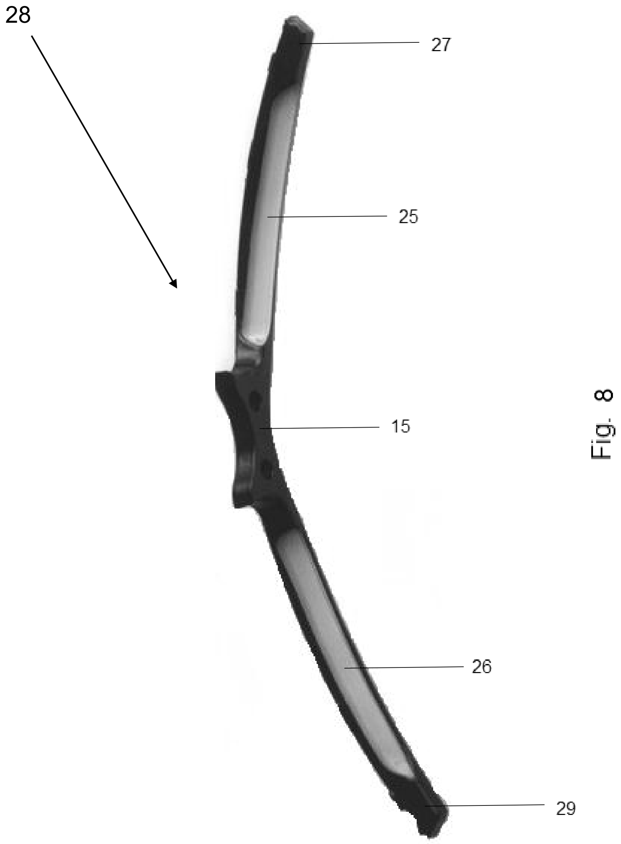

Figure 7 shows an embodiment of the glasses of the invention, wherein the nose frame part 15 and the light guiding elements 25 and 26 form a unit 28 with a nose frame part and two light guiding elements. Figure 8 shows such a unit 28 with a nose frame part and two light guiding elements, according to the invention, wherein the separate parts building up unit 28, namely the nose frame part 15 and the light guiding elements 25 and 26, are indicated. In unit 28 with a nose frame part and two light guiding elements the nose frame part 15 connects the light guiding elements 25 and 26 such that a single part is formed. The application of the unit 28 with a nose frame part and two light guiding elements in a glasses of the invention alternative to a nose frame part 15 and light guiding elements 25 and 26, is optional according to the invention.

The sides of the support frame 9 that are facing the eyes of the wearer and that are positioned below the light guiding elements 25, 26, such as for example the nose frame side portion 17, reflect the artificial light that is coming indirectly from the light guiding elements 25, 26, to the eye of the user. The other sides of the support frame 9 located below the light guiding elements 25, 26, that are facing the eye when in use, can be provided in the same manner as the nose frame side portion 17, such that the amount of light emitted to the eyes is increased. These sides of support frame 9 comprise support frame sides 35, 36 facing the eyes, which extents in the glasses 1 between the nose frame side portion 17 and the support frame upper part 23. The reflecting sides of the support frame 9 can comprise inner surfaces that surround the spectacle glass 3a, 3b, such as the inner surface 16 of the nose frame part and the other inner surfaces 38, 39 of the support frame 9 located below the light guiding elements 25, 26.

In addition, the glasses 1 is provided with a soft lining 40 for enhancing the comfort of the wearer of the glasses 1. Then, it is possible to provide at least the sides of the lining that are facing the eyes as reflecting lining in order to reflect the incident indirect light from the light guiding elements 25, 26 on said sides of the lining, when in use, to the eyes.

The light reflecting parts of the glasses 1, such as the sides of the support frame 9 and/or the lining, reflect the incident light for at least 50%, preferably for more than 85%.

With the glasses of the invention it is possible to project 1 lux at minimum and 6 lux at maximum at the level of the retina, for example between 1 lux and 4 lux, or for example 4 lux to 6 lux, and 36 lux at minimum and 50 lux at maximum at the level of the eye, for example about 40 lux, or alternatively 8 lux at minimum and 10 lux at maximum at the level of the eye. A light intensity of between 4 lux and 6 lux at the level of the retina and between 36 lux and 50 lux at the level of the eye is essential for executing an effective artificial light treatment.

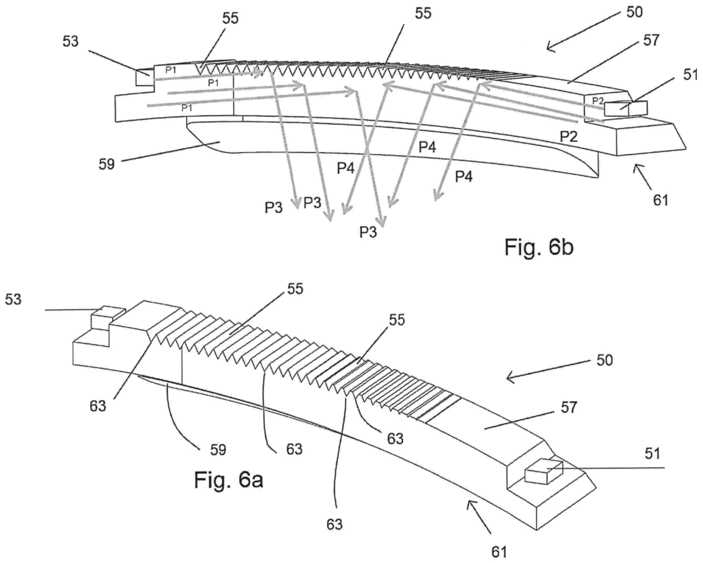

The Figures 6A, 6B show another embodiment of the light guiding element 50. This light guiding element 50 can be assembled in the support frame upper part 23 of the glasses 1 shown in Figures 1-5 at the same position as the light guiding element 25, 26.

The elongated light guiding element 50 is provided with a first LED 51, as well as with a second LED 53. For executing a light treatment, with the light guiding element 50 no further light sources are required in the glasses (not shown). The elongated light guiding element 50 is provided with an upper side 57 partly provided with notches 55, as well as a portion 59 protruding at the bottom side 61.

The portions of upper side 57 located nearby the LEDs 51, 53 are flat without notches 55. The notches 55 are provided between the flat portions of the upper side. The portion with notches 55 is larger than the two flat end-portions together. The notches 55 extend transversely to the longitudinal direction of the elongated light guiding element 50. The cross-section of each notch 55 is triangular. The notches 55 become evenly deeper toward the second LED 53, while the distance between the centers 63 of the consecutive notches remains constant.

The LEDs 51, 53 emit the artificial light substantially laterally in the light guiding element 50 in the direction indicated by the arrows P1 and P2 to the notches 55. The artificial light is reflected by the notches 55 in the direction indicated by arrows P3 and P4 to the eye of a user.

In one embodiment, the surface of the flat portion 59 of the elongated light guiding element that faces downwardly (bottom side 61), when the glasses are worn, is only transparent for photons at one or more discrete portions of said surface. This way, photons delivered by the photon source comprised by the light source in the glasses of the invention, can be directed to pre-determined portions of the eye and to predetermined portions of the retina of the eye of the wearer of the glasses. In an alternative embodiment, the complete surface of the flat portion 59 of the elongated light guiding element that faces downwardly (bottom side 61), when the glasses are worn, is transparent for photons. This way, the photons emitted by the photon source comprised by the light source in the glasses of the invention are delivered efficiently to the eye and the retina of the wearer of the glasses over the whole length of the light guiding element located above the eye of the wearer of the glasses, with less loss of energy (photons) when compared to a partially non-transparent light guiding element.

The light guiding element 50 is preferably made of polycarbonate (e.g., ALCOM pwl 10 / 1.1 WT1302-05LB). It is possible to add colored or light reflective pigments to the material so that the light can be reflected to the eyes diffusely.

The frame 5 is provided with a control unit such as a circuit board (PCB) and a battery. The battery that is comprised by the glasses of the invention is for example a rechargeable lithium ion polymer battery known in the art, according to the invention. Optionally, the frame 5 may be provided with a memory storage. These components may be located in the frame side portions 12, 14 or in the frame legs 7a, 7b. Further, the frame may include a communication device (not shown) such as, for example, a USB port such as a microUSB port, for providing a wired connection and / or, for example, a Bluetooth chip for providing a wireless connection.

The glasses 1 may be part of a system further comprising an external device comprising a processor as well as a computer program which, in use, causes the processor to control the LEDs in the glasses in accordance with a predetermined program.

It is possible that the glasses is designed so that the support frame of the frame only partially encloses the perimeter of the spectacle glasses.

It is also possible that at least portions of the surface of the support frame side facing the wearer's eye are designed such that the light to be reflected is diffused. Also, portions of the support frame facing the wearer's eye can be curved such that the light to be reflected is directed to a position in the eye. Further, the side of the support frame facing the wearer's eye may be provided with whitening agents.

The LEDs for emitting the blue light and UV light may be provided with filters for filtering the eye-damaging part of the light spectrum of the light emitted by the light source, such as, for example, UV radiation having a wavelength between 10 and 400 nanometer. These filters can also be provided elsewhere in the glasses to prevent a malicious part of the light emitted with the light source from reaching the eye. Preferably, the LEDs for emitting blue light do not emit UV light, according to the invention. This way, exposing the eyes of the wearer of a glasses of the invention to harmful UV light emitted by the glasses is prevented. Preferably, the blue light emitted by the LEDs in the glasses of the invention has a wavelength of between 465 nm and 475 nm, +/- 10 nm. More preferably, the blue light emitted by the LEDs in the glasses of the invention has a wavelength of about 468 nm +/- 10 nm, said LED providing about 4 lux to 6 lux at the level of the retina of the eye of the wearer of the glasses, and providing about 36 lux to 50 lux at the level of the lens of the eye of the wearer of the glasses, according to the invention. In one embodiment, the glasses of the invention are provided with two to four blue light emitting LEDs, preferably four LEDs, wherein said LEDs for emission of blue light have an emission peak wavelength of about 468 nm and a spectral line half width of about 25 nm. In one embodiment, said four LEDs comprised by the glasses of the invention comprise a photon source capable of delivering 36 lux to 50 lux, preferably about 40 lux, at the level of the eye of the wearer of the glasses, and capable of delivering 4 lux to 6 lux at the level of the retina of the eye of said wearer of the glasses, and capable of emitting photons with an emission peak wavelength of 468 nm. Typically, a LED applied in the glasses of the invention is a LED comprising as the photon source a p/n semiconductor material consisting of a p-layer and an n-layer of InGaN (*e.g.* product *LTST-108TBKT*; Lite-on Technology Corporation). In the Table, below, further examples of LEDs applicable in the glasses of the invention are listed. According to the invention, a blue light emitting LED with an emission peak wavelength of about 468 nm and a relatively small full width at half maximum spectral band width of about 18 nm to 37 nm, preferably about 24 nm, is preferred.

| **TABLE: LEDs applicable in the glasses of the invention** | | |
|---|---|---|
| **LED (supplier)** | **Emission peak wavelength (nm)** | **λ0,5, the Full Width at Half Maximum spectral bandwidth (nm)** |
| ASMT-AL31 (Broadcom - Avago) | 450.0 | 19.5 |
| ASMT-JL-31-NPQ01 (Broadcom - Avago) | 452.6 | 24.0 |
| 21.00.01B (XLED, Italy) | 454.7 | 28.0 |
| ASMT-JL11-NM (Broadcom - Avago) | 456.5 | 25.7 |
| ASMT-JB-31-NMP01 (Broadcom - Avago) | 459.9 | 24.0 |
| LB-W5SN-GYHZ-25 (Osram opto semiconductors) | 460.0 | 32.6 |
| LB-T673-L2P1-35 (Osram opto semiconductors) | 462.2 | 20.0 |
| LT-T67C-K1M2-35 | 465.0 | n.d. |
| VAOL-S2SB4 with source color InGaN (VCC optoelectronics) | 468 (minimum dominant λ is 464,0 nm, maximum dominant λ is 472,0 nm) | 35 |
| LTST-C194TBKT with source color InGaN (Lite-on technology corp.) | 468 (minimum dominant λ is 460,0 nm, maximum dominant λ is 475,0 nm) | 25 |
| LV-W5AM-JYKY-25 (Osram opto semiconductors) | 501.7 | 28.0 |

Alternatively to the application of blue light emitting LEDs, different light sources than such LEDs are also applicable in the glasses of the invention. For example, a photon source such as a blue-light fluorescent dopant comprised by an organic LED (OLED) light source or a photon source such as a deep-blue light phosphorescent dopant comprised by a phosphorescent organic LED (PHOLED) light source, wherein said photon source is capable of emitting blue light, is also applicable for application in the light sources comprised by the glasses of the invention.

Such a photon source comprised by an OLED light source is for example a blue fluorescent dopant for example with an emission peak wavelength of about 468 nm, such as for example 3-(9*H-*[3,9'-bicarbazol]-9-yl)-9*H-*xanthen-9-one-dibenzo[*b,d*]furan-2,8-diylbis(diphenylphosphine oxide) (CCX-I:PPF), according to the invention. Such a photon source comprised by a PHOLED light source is for example a blue phosphorescent dopant for example with an emission peak wavelength of about 468 nm, such as for example an organometallic-iridium(III) complex and benzo(e)pyrene. Of course, many more blue fluorescent dopants and blue phosphorescent dopants for application in blue light emitting OLED light sources and PHOLED light sources, respectively, are known in the art, and are equally applicable in a light source comprised by the glasses of the invention, as long as the application of such photon sources in the glasses of the invention provide for glasses capable of delivering about 40 lux at the level of the eye of the wearer of the glasses and capable of delivering about 4 lux to 6 lux at the level of the retina of the wearer of the glasses, according to the invention.

It is further possible to provide the glasses with at least a light source integrated centrally in the frame, for direct and / or indirect delivering light to the eyes substantially from above.

It has been found by the inventors that photons are most efficiently provided to the retina of the eye of a wearer of the glasses of the invention, when photons are delivered essentially from above the eye through the lens of the eye on the retina in the eye. Without wishing to be bound by theory, this observed highest efficiency is achieved since the light sensitive nerve cells of the retina (intrinsic photosensitive retinal ganglion cells) are predominantly located at the bottom side of the eye (when the wearer of the glasses is in upright position). Furthermore, it has been found by the current inventors that melatonin production is efficiently suppressed in the host wearing the glasses of the invention when the at least one light source of the glasses delivers about 40 lux at the level of the lens of the eye of the wearer, according to the invention.

Preferably, the glasses of the invention comprises as a light source four blue light emitting LEDs, each such a LED having a power of about 0,1 Watt. Preferably, such LEDs comprise a photon source capable of emitting photons with an emission peak wavelength of about 468 nm.

The frame 5 can be provided with two hinging frame legs. In the position of use of the glasses 1 with unfolded hinging frame legs, the glasses 1 can be worn by a person, such that each spectacle glass 3a, 3b is positioned in front of one of the two eyes of a person aided by frame 5 for positioning on the ears and nose of the person. By hinging the frame legs towards each other in the direction of the spectacle glasses 3a, 3b, the glasses 1 is brought from the unfolded state to a compact folded state, such that it is stored, for example, in a glasses case (not shown).

The glasses case and the glasses can be provided with Qi technology such that the battery of the glasses is wirelessly chargeable. Furthermore, the glasses case itself can be provided with a battery such that the glasses case without cables can wirelessly charge the battery of the glasses by using the battery of the glasses case.

An alternative glasses can be provided with two transparent spectacle glasses enclosed in a frame, wherein each spectacle glass is positioned in front of an eye of a person by a frame for positioning on the ears and the nose of said person, the glasses further provided with at least one light source integrated in the frame, wherein above the spectacle glass the frame is provided with at least a light guiding element for indirect delivery of light substantially from above the spectacle glass to the eyes, the light originating from at least one light source. The alternative glasses comprises a light guiding element with notches as herein described and as shown in the Figures. Optionally, the alternative glasses may comprise a frame with a nose frame part, which nose frame part at, at least, a nose frame side portion facing the eye, reflects to the eye at least partially the direct or indirect incident light thereon originating from the light source.

### Case studies

- Glasses of the invention were successfully used for beating jetlag by 120 traveling athletes.
- Glasses of the invention were also successfully used by athletes for shifting sleep/wake cycles because of early morning or late evening matches so the optimal peak-performance moments were synchronized with the time of the day.
- Glasses of the invention were tested in two companies having employees working during night time, for improving shift work conditions. Alertness improved in most cases and fatigue was less pronounced, showing the effectiveness of the use of the glasses, according to the invention.
- Glasses according to the invention are successfully used by many subjects to diminish the symptoms of fatigue and sadness caused by the change of seasons (winter blues).

## Claims

1. An applicator for use in a method of treating a neurodegenerative disease in a patient in need thereof, wherein the applicator is provided with at least one light source for delivery of photons to an eye, said light source comprising a photon source capable of emitting photons with a photon energy of between 2,82 eV and 2,43 eV, wherein photons emitted by said photon source are delivered on the retina of an eye of a patient subjected to said treatment.

2. The applicator for use according to claim 1, wherein said applicator is a glasses (1) provided with a support frame (9) for positioning on the ears and the nose of a person;
the support frame (9) enclosing at least partially two perimeters for receiving two transparent spectacle glasses (3a, 3b), and
at least one light source of claim 1 integrated in the support frame (9) for emitting light,
wherein the support frame portion (23) located above the perimeters comprises at least one light guiding element (25, 26, 50) adapted to indirectly deliver light originating from the at least one light source substantially from above the perimeters to the eyes, wherein the support frame (9) comprises an inner surface (16) that surrounds at least partially each of the perimeters, wherein the portion (38, 39) of the inner surfaces (16) that is located below the at least one light guiding element (25, 26, 50) are configured to reflect the artificial light that is coming indirectly from the at least one light guiding element (25, 26, 50) onto the eye of the person,
wherein the glasses are constructed such that during light treatment:
each perimeter for receiving two spectacle glasses (3a, 3b) is positioned in front of an eye of the person,
the portion (38, 39) of the inner surfaces of the support frame that surrounds at least partially each of the perimeters for receiving two spectacle glasses (3a, 3b) and that is located below the at least one light guiding element (25, 26, 50) at least partially reflects to the eye direct or indirect incident light originating from the light source, and
the photons are directly and/or indirectly delivered to the eyes substantially from above the perimeter.

3. The applicator for use according to claim 2, wherein the at least one light guiding element (25, 26, 50) located in the support frame portion (23) above the perimeters for receiving spectacle glass (3a, 3b) is provided with an upper side (57) having notches (55).

4. The applicator for use according to claim 2 or 3, wherein each notch (55) extends transversely to the longitudinal direction of the light guiding element (25, 26, 50).

5. The applicator for use according to any one of the claims 2 - 4, wherein the notches (55) in the direction of the nose frame part become deeper.

6. The applicator for use according to any one of the claims 2 - 5, wherein the notches (55) in the direction of the nose frame part become evenly deeper.

7. The applicator for use according to any one of the claims 2 - 6, wherein the distances between the centers (63) of the notches (55) are constant.

8. The applicator for use according to any one of the claims 2 - 7, wherein the support frame (9) encloses at least half the perimeter for receiving the spectacle glasses (3a, 3b), wherein with a worn glasses the portion of the support frame positioned lower than the support frame upper portion (57) at, at least, a side of the support frame (9) that is directed to the eye, at least partly reflects to the eye the incident light thereon directly or indirectly originating from the light source, wherein the eye-directed side of the support frame (9) at least partially reflects the incident light originating directly or indirectly from the light source by at least 50%, or reflects more than 85%, optionally wherein the surface of at least the eye-directed side portion is at least partially configured to reflect the light to be reflected diffusely to the eye.

9. The applicator for use according to any one of the claims 2 - 8, wherein the at least eye-directed side portion is at least partly provided with whitening agents.

10. The applicator for use according to any one of the claims 2 - 9, wherein the light source is at least one light-emitting diode (51, 53), which light-emitting diode (51, 53) can emit blue light, wherein the blue light has a wavelength of 464 - 480 nanometer.

11. The applicator for use according to any one of the claims 2 - 10, wherein 36-50 lux is deliverable at retinal level of the eye of the person with the aid of the at least one light source.

12. The applicator for use according to any one of the claims 2 - 11, wherein the support frame encloses at least partially two perimeters receiving two transparent spectacle glasses (3a, 3b).

13. The applicator for use according to any one of the claims 1 - 12, wherein the neurodegenerative disease is Parkinson's disease.

14. The applicator for use according to any one of the claims 1 - 13, wherein the method of treating comprises treating, preventing or relieving at least one of the symptoms of Parkinson's Disease, preferably selected from bradykinesia, pain, akinesia, sleep disturbance, depressed mood, sadness, melatonin levels during daytime and dystonia.

15. The applicator for use according to any one of the claims 1 -14, wherein photons emitted by the photon source are delivered at the retina of said patient at least once daily for between about 15 minutes and 60 minutes, or for about 30 minutes, optionally wherein photons emitted by the photon source are delivered at the retina of said patient once daily for about 30 minutes, the delivery of the photons starting after between about 0 minutes and four hours of the time of awakening after overnight sleep, or between 30 minutes and 2 hours,
optionally wherein the patient is treated for a time period of at least one week, or at least one month, or at least one year, or for the rest of the lifespan of the patient.
